(19) Europäisches Patentamt
European Patent Office
Office européen des brevets

(11) **EP 4 080 202 A1**

(12) **EUROPEAN PATENT APPLICATION**

(43) Date of publication:
**26.10.2022 Bulletin 2022/43**

(21) Application number: **21169651.3**

(22) Date of filing: **21.04.2021**

(51) International Patent Classification (IPC):
**G01N 27/12** (2006.01)    **G01N 33/00** (2006.01)

(52) Cooperative Patent Classification (CPC):
**G01N 27/12; G01N 33/0006**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Infineon Technologies AG
85579 Neubiberg (DE)**

(72) Inventors:
• **Schober, Sebastian
80469 München (DE)**
• **Carbonelli, Cecilia
81477 München (DE)**

(74) Representative: **Stöckeler, Ferdinand et al
Schoppe, Zimmermann, Stöckeler
Zinkler, Schenk & Partner mbB
Patentanwälte
Radlkoferstrasse 2
81373 München (DE)**

(54) **GAS SENSING DEVICE AND METHOD FOR DETERMINING A CALIBRATED MEASUREMENT VALUE OF A CONCENTRATION OF A TARGET GAS**

(57)     A sensing device for sensing a target gas comprises a measurement module configured for providing measurement information about a measurement of the concentration. The sensing device further comprises a signal calibration module which is configured for using a machine learning model for determining, on the basis of the measurement information, a calibrated measurement value of the concentration. The signal calibration module is further configured for determining a feedback feature using the calibrated measurement value. The signal calibration module is further configured for using the machine learning model for determining a subsequent calibrated measurement value on the basis of subsequent measurement information about a subsequent measurement of the concentration and on the basis of the feedback feature.

Fig. 2

**Description**

Examples of the present disclosure relate to a sensing device for sensing a concentration of a target gas. Further examples relate to a method for determining a calibrated measurement value of a concentration of a target gas. In particular, some examples of the present disclosure relate to chemoresistive gas sensing devices and methods for calibrating measurement signals of chemoresistive gas sensing devices. Some examples may relate to chemoresistive multi-gas sensing devices. Some examples of the present disclosure relate to a feedback loop architecture for graphene-based gas sensor drift compensation.

Gas sensors are used for sensing a concentration of one or more target gases of the gas sensor in the environment of the gas sensor. Gas sensors usually measure a signal which depends on the concentration of the target gas and estimate a value for the concentration from the measured signal. As conditions, under which the signal is measured, and/or a state of the gas sensor may change over time, the relation between the measured signal and the true concentration of the target gas may vary accordingly. This sensor drift makes it difficult to estimate the true concentration. Examples of gas sensing devices are chemoresistive gas sensing devices which rely on the principle, that molecules of the target gas adsorb at a sensing surface of the sensing device, resulting in a modification of an electrical resistivity, the latter being measured so as to obtain a measurement signal. In particular, multi-gas sensor arrays, which may comprise, for example, a plurality of chemoresistive sensors, are an efficient and cost saving way for measuring gasses and evaluating air quality. Different signals produced by chemical processes between the sensor materials and the air are measured and then translated into gas concentrations using algorithms which often make use of artificial intelligence to differentiate between gasses. One major problem of this technical process is the observation that the signal values from the sensor are often not absolute, for example due to the above described sensor drift. This makes it hard for the algorithms to estimate the real gas concentrations, since there is no steady baseline to compare the current signal value to.

Accordingly, a concept for calibrating measurement information of a gas sensing device is desirable, which concept provides for a reliable drift compensation.

Examples of the present disclosure rely on the idea of using a machine learning model, such as a neural network, for determining calibrated measurement values of a concentration of a target gas. The idea includes to determine a feedback feature on the basis of one or more of the determined calibrated measurement values and to use the feedback feature in the determination of a subsequent calibrated measurement value. Consequently, the feedback feature may represent a temporal evaluation of measurement information, based on which the calibrated measurement values are determined, and may therefore be indicative of a drift of the sensing device.

Examples of the present disclosure provide a sensing device for sensing a concentration of a target gas. The sensing device comprises a measurement module configured for providing measurement information about a measurement of the concentration. The sensing device further comprises a signal calibration module. The signal calibration module is configured for using a machine learning model for determining, on the basis of the measurement information, a calibrated measurement value of the concentration. The signal calibration module is further configured for determining a feedback feature using the calibrated measurement value. The signal calibration module is configured for using the machine learning model for determining a subsequent calibrated measurement value on the basis of subsequent measurement information about a subsequent measurement of the concentration and on the basis of the feedback feature.

Further examples of the present disclosure provide a method for determining a calibrated measurement value of a concentration of a target gas. The method comprises a step of obtaining measurement information independent on the concentration of the target gas. The method further comprises a step of using a machine learning model, such as a neural network, for determining, on the basis of the measurement information, a calibrated measurement value of the concentration. The method comprises a step of determining a feedback feature using the calibrated measurement value. The method further comprises a step of using the machine learning model for determining a subsequent calibrated measurement value on the basis of the measurement information and on the basis of the feedback feature.

Examples and advantageous implementations of the present disclosure are described in more detail below with respect to the figures, among which:

Fig. 1        illustrates an example of a sensing module,

Fig. 2        illustrates an example of a sensing device,

Fig. 3        shows a diagram with examples of evolutions of the feedback feature,

Fig. 4        illustrates a block diagram of an example of a feedback loop,

Figs. 5A, B   illustrate examples of the calibration unit,

Figs. 6A, B   illustrate examples of the signal calibration module,

Fig. 7        shows diagrams demonstrating the accuracy of an example of the determination of the calibrated measurement values,

Fig. 8    illustrates test set scores of an example of the determination of the calibrated measurement values,

Fig. 9    illustrates a flow chart of an example of a method for determining a calibrated measurement value.

In the following, examples are discussed in detail, however, it should be appreciated that the examples provide many applicable concepts that can be embodied in a wide variety of sensing applications. The specific examples discussed are merely illustrative of specific ways to implement and use the present concept, and do not limit the scope of the examples. In the following description, a plurality of details is set forth to provide a more thorough explanation of examples of the disclosure. However, it will be apparent to one skilled in the art that other examples may be practiced without these specific details. In other instances, well-known structures and devices are shown in form of a block diagram rather than in detail in order to avoid obscuring examples described herein. In addition, features of the different examples described herein may be combined with each other, unless specifically noted otherwise.

In the following description of examples, the same or similar elements or elements that have the same functionality are provided with the same reference sign or are identified with the same name, and a repeated description of elements provided with the same reference number or being identified with the same name is typically omitted. Hence, descriptions provided for elements having the same or similar reference numbers or being identified with the same names are mutually exchangeable or may be applied to one another in the different examples.

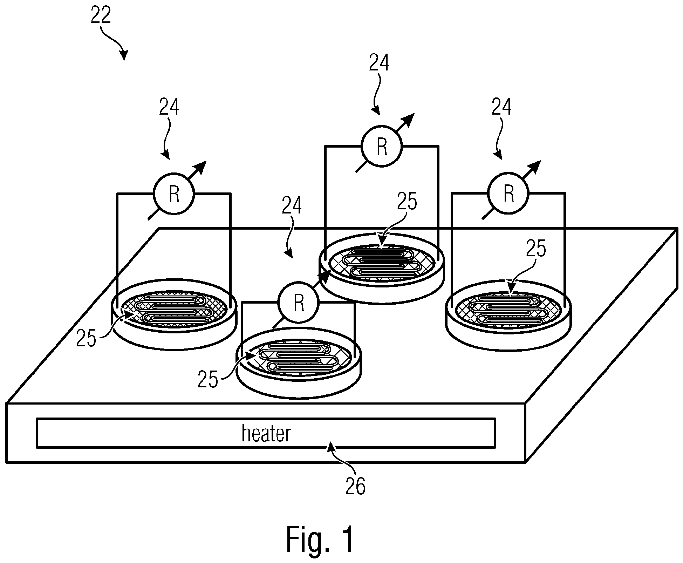

Fig. 1 illustrates a sensing module 22 according to an example of the present disclosure. The sensing module 22 comprises a plurality of sensing units 24, each of which is sensitive to a target gas out of a plurality of target gasses. For example, each of the sensing units 24 may be sensitive to a different target gas. Alternatively, one or more of the sensing units 24 may be sensitive to the same target gas, so as to provide redundant measurement signals. Each of the sensing units 24 comprises a sensing surface area 25 comprising a sensing material which is sensitive to the target gas of the respective sensing unit 24. Each of the sensing units 24 is configured for providing a measurement signal which depends on a concentration of the target gas of the respective sensing unit 24 in the environment of the sensing module 22. It should be noted that an individual one of the sensing units 24 may be sensitive to multiple target gasses, wherein the sensitivity of the sensing unit 24 may be different for the different target gasses. In some examples, the sensing module 22 may comprise a single one of the sensing units 24, in other examples a plurality.

During exposure of the sensing module 22 to a gas, the target gas or another gas, molecules of the gas may absorb at the sensing surface area 25 of one or more of the sensing units 25, such influencing the measurement signal provided by the sensing unit. As desorption of the gas molecules from the sensing surface area may be slow compared to the adsorption, a high number of gas molecules may accumulate at the sensing surface area 25, resulting in a decrease of the sensitivity of the sensing unit 24. The sensing module 22 may optionally comprise a heater 26, for example, one heater for all of the sensing units 24, or individual heaters for the sensing units 24, By heating the sensing surface areas 25, a desorption of the gas molecules from the sensing surface area 25 may be accelerated.

According to examples, the sensing units 24 are carbon-based, that is, the sensing material of the sensing surface areas 25 the sensing units 24 may comprise a carbon material, such as graphene or graphite.

An example of the of the sensing module of Fig. 1 is a graphene-based multi-gas sensor array which consists of four graphene-based sensing units 24, where the base material (i.e. the sensing material of the respective sensing unit, which is exposed to the environment in which the concentration of the target gas is to be measured) is functionalized with different chemicals (e.g. Pd, Pt, and $M_nO_2$) for dissimilar selectivity. The interaction between graphene sheets and adsorbed gas analytes would influence the electronic structure of the material, resulting in altered charge carrier concentrations and changed electrical conductances. Meanwhile, due to different sensitivity towards various gas molecules, resistances of the sensors also change in disparate patterns, making it possible to analyze complicated gas mixtures with one single sensor array. Each sensor in the array has a heating element whose temperature is being pulsed between a recover phase temperature and a sense phase temperature.

In general, for adsoption-based sensing devices, such as the sensing units 24, during the measurement process the time needed for the desorption of the gas molecules from the sensor surface tends to be much longer than the time of adsorption. This can lead to a downwards drift behavior of the signal which means that the sensor baseline changes over time and makes it much harder to predict the target gas concentration with a reasonable accuracy. Algorithms can help to get rid of such artifacts and changing baselines by taking previous gas exposure of the sensor surface into account and compensate accordingly for short-term drift that results from this exposure.

An aim of examples of the disclosure is to suppress this short-term drift in the output signal of the sensor by recycling previous predictions (of concentration values) and integrating them into the regression process (of prediction the concentration values based on the measurement values of the sensing module 22). In order to achieve this, a so-called Feedback-Loop architecture has been designed and invented for a neural network, or more general, for a machine learning model, which may be employed in the determination of the concentration values.

Examples of the sensing module 22, e.g. the graphene-based multi-gas sensor array, may be employed in a sensing device 10 which is described in the following.

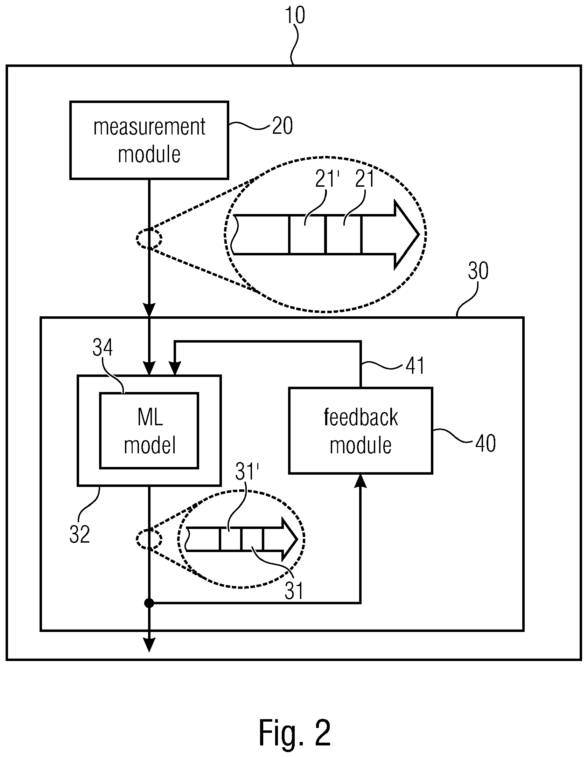

Fig. 2 illustrates a sensing device 10 according to an example of the present disclosure. The sensing device 10 is configured for sensing a concentration of a target gas in an environment of the sensing device 10. For example, the target gas may be one of $NO_2$, $O_3$, $NH_3$. In general, the target gas may refer to any substance in the gas phase, so that the concentration of the target gas may also refer to a relative humidity of water vapor in the environment of the sensing device 10. The sensing device 10 comprises a measurement module 20. The measurement module 20 provides measurement information 21 about a measurement of the concentration. The sensing device 10 further comprises a signal calibration module 30, e.g. implemented by a signal processor. The signal calibration module 30 comprises a calibration unit 32, which uses a machine learning model 34 for determining, on the basis of the measurement information 21, a calibrated measurement value 31 of the concentration. The signal calibration module 30 further comprises a feedback module 40 which determines a feedback feature 41 using the calibrated measurement value 31. The calibration unit 32 uses the machine learning model 34 for determining a subsequent calibrated measurement value 31' on the basis of subsequent measurement information 21' about a subsequent measurement of the concentration and on the basis of the feedback feature 41.

For example, the measurement may refer to an evaluation of a measurement signal at an instance of time of the measurement. The measurement module 20 may, for example, obtain one or more measurement signals. The measurement module 20 may sample, or evaluate, the one or more measurement signals at the instance of time of the measurement, so as to obtain the measurement information 21 about the measurement. For example, the measurement information 21 may comprise one or more of a signal level, a derivative, a second derivative, and an energy vector of the one or more measurement signals at the time instance of the measurement to which the measurement information 21 refers. Accordingly, the measurement module 20 may provide the subsequent measurement information 21' about the subsequent measurement by evaluating the one or more measurement signals at a subsequent time instance.

For example, the calibrated measurement value 31 is an estimation (also referred to as a prediction) of the real concentration of the target gas at the time instance of the measurement to which the measurement information 21 refers.

The calibration unit 32 uses the feedback feature 41, which is determined using the calibrated measurement value 31, for determining the subsequent calibrated measurement value 31'. In using the feedback feature 41, the calibration unit 32 may use information about a previous calibrated measurement value, the calibrated measurement value 31, in the determination of the calibrated measurement value 31'. Thus, the feedback feature 41 may be representative of a temporal evolution of the calibrated measurement values 31 over a sequence of measurements of the concentration. The feedback feature 41 may such enable the calibration unit 32 to account for a drift in the determination of the measurement information by measurement module 20, such as, for example, a saturation of the sensing surface area 25 of one of the sensing units 24. In other words, the feedback feature 41 may enable the calibration unit 32 to compensate a drift between the calibrated measurement values and the real values of the concentration of the target gas. As the feedback feature 41 is based on the at least one calibrated measurement value 31 determined by the sensing device 10, the disclosed concept may enable drift compensation independent from reference information provided to the sensing device 10 over the air. Thus, the concept may, for example, be employed to sensing devices which are not permanently connected to an instance, such as a server, providing reference information to the sensing device 10. Further, as the feedback feature 41 is based on the calibrated measurement value 31, it may account for both, exterior environment effects and internal behavior of the sensor, which both may result in a sensor drift. Thus, the disclosed algorithm is particularly flexible, that is, for example, the algorithm may account for various drift behaviors, which may vary in dependence on the environment, in which the sensing device 10 is deployed. Thus, the sensing device may be deployed in different geographic regions, for example, using the same calibration procedure.

Using the feedback feature 41 for the determination on the subsequent calibrated measurement value 31' further has the advantage that the feedback feature 41 may require less memory compared to a method in which the measurement information 21 itself would be buffered for usage in the determination of the subsequent calibrated measurement value 31'. As described above, the measurement information 21, 21' may comprise a plurality of values representing features of one or more measurement signals. Thus, storing the feedback feature 41 for usage in the determination of the subsequent calibrated measurement value 31' may save memory.

In examples, the measurement module 20 comprises one or more chemoresistive sensing units, e.g. one or more of the sensing units 24 as described with respect to Fig. 1, each of the sensing units providing a measurement signal. The sensing units may be carbon-based or graphene-based. For example, the measurement module 20 may comprise the sensing module 22. Determining the feedback feature 41 for the determination of the calibrated measurement value 31' on the basis of the previous calibrated measurement value 31 may, for example, be particularly beneficial for short-term drift compensation. A short-term drift may be a drift which varies over time, e.g. a reversible drift. For example, the case of concentration related sensitivity loss of adsorption based sensing units (chemoresistive sensing units) as described with respect to Fig. 1 may be an example of a short-term drift, as the drift may decrease with desorption of the molecules. In examples in which the measurement module 20 comprises the sensing module 22 of Fig. 1, in particular, in examples in which the sensing module 22 comprises carbon-based chemoresistive sensing units 24, the feedback feature may, for example, be representative of a short-term signal drift of this type of graphene sensor. In this case, the feedback feature 41 may reflect the occupancy of the one or more sensing surface areas 25 after gas adsorption. In other words, the usage of the feedback loop architecture, as implemented by the feedback feature 41, may enhance the reliability of the chemoresistive, e.g. graphene-based, gas sensing device, e.g. a multi-gas sensor, by giving it one or more features which are related to the internal short-term signal drift of this type of (graphene-based) sensing device. This is accomplished by leveraging one or more previous predictions on gas concentration and feeding back the feedback feature 41 which may reflect the occupancy of the sensor after gas adsorption.

Examples of the sensing device 10 of the present disclosure may be outdoor air quality sensors or other multi-gas sensors. For example, the signal calibration unit 30 may be provided as part of an API package for multi-gas sensors.

According to examples, the feedback module 40 is configured for determining the feedback feature 41 for the determination of the subsequent calibrated measurement value 31' on the basis of the calibrated measurement value 31 and on the basis of one or more previously determined calibrated measurement values.

The previously determined calibrated measurement values may be determined on the basis of previous measurement information about previous measurements relative to the measurement of the measurement information 21. In other words, the feedback feature 41 for the determination of the subsequent calibrated measurement value 31' on the basis of a plurality of calibrated measurement values determined previously to the subsequent calibrated measurement value 31', i.e., based on measurement information about a plurality of previous measurements of the concentration (that is, previous to the measurement to which the subsequent calibrated measurement value 31' to be determined is related).

Being based on a plurality of previously determined calibrated measurement values, the feedback feature 41 may accurately represent a temporal evolution of the calibrated measurement values, such allowing the machine learning module 34 for an accurate drift compensation.

According to examples, the feedback module 40 is configured for determined the feedback feature 41 for the determination of the subsequent calibrated measurement value 31' by updating a previously determined feedback feature using the calibrated measurement value 31. The previously determined feedback feature is determined on the basis of previously calibrated measurement values.

For example, updating the previous feedback feature may include to combine the previous feedback with the calibrated measurement value 31. The previous feedback feature may be a feedback feature used for determining the calibrated measurement value 31. Thus, updating the previous feedback feature allows for determining the feedback feature 41 so that it represents an evolution of the calibrated measurement values over a sequence of measurements, such providing information on a temporal evolution of the calibrated measurement value over a plurality of measurements. Updating the feedback feature 41 may further allow for an implementation of the feedback feature which requires a particularly low amount of memory.

For example, the measurement information 21 and the subsequent measurement information 21' result from a sequence of measurements of the concentration. In these examples, the calibrated measurement value 31 and the subsequent measurement value 31' are part of a sequence of calibrated measurement values, and the calibration unit 32 is configured for determining the calibrated measurement values on the basis of measurement information resulting from respective measurements of the sequence of measurements. According to these examples, the calibration unit 32 is configured for determining the feedback feature 41 for the determination of one (the current one) of the calibrated measurement values by recursively updating the feedback feature 41 using the previous one of the calibrated measurement values. For example, the calibration unit 32 is configured for determining each of the calibrated measurement values of the sequence of measurement values on the basis of the measurement information of one or the measurements of the sequence of measurements.

In other words, the feedback feature 41 may be an implementation of a self-reassigning loop architecture. The feedback-loop may link the previous outputs of the sensor to the successively updated feedback feature 41 and such provides drift-related information into the algorithm. It is noted, that the calibrated measurement value 31 and the subsequent measurement value 31' may refer to any two subsequent calibrated measurement values of the sequence of calibrated measurement values. For example, the feedback feature 41 may, for a first measurement of the sequence, be initialized using an initialization value, e.g. a default value.

According to examples, the feedback module 40 is configured for determining the feedback feature 41 on the basis of a weighted sum of the calibrated measurement values and one or more previously determined calibrated measurement values.

Determining the feedback feature 41 on the basis of a weighted sum allows for weighting respective contributions of the calibrated measurement value 31 and the one or more previously determined calibrated measurement values to the feedback feature 41 according to lengths of time intervals between the corresponding measurements of the calibrated measurement value 31 and the one or more previously determined calibrated measurement values and the subsequent measurement for the calibration of which the feedback feature 41 will be used. Further, a selection of the weights for the weighted sum allows to adapt the determination of the feedback feature 41 to the process which potentially induces the drift effect which is to be compensated by the feedback feature 41.

According to examples, the measurement module 20 comprises at least one chemoresistive sensing unit 24 which is sensitive to the target gas, for example, the measurement model 20 may comprise the sensing module 22 of Fig. 1. According to these examples, the weights of the weighted sum may be adapted to a desorption rate of molecules of the target gas adsorbed at the surface region 25 of the sensing unit 25.

As the sensitivity of the sensing unit 24 may depend on a number of adsorbed gas molecules at the sensing surface area 25 of the sensing unit 24, the concentration of the target gas at time instances before a measurement may influence the measurement signal measured during the measurement. Adapting the weights of the weighted sum to the desorption rate may therefore allow to determine the feedback feature 41 so that the feedback feature 41 is representative of an occupancy of adsorption sites of the sensing surface area 25 at the time instance of the measurement for the calibration of which the feedback feature 41 is to be used.

An example of an implementation for the determination 40 of the feedback feature 41, which needs only a small amount of computational operations would be an online computation described by:

$$L_t = d \cdot L_{t-1} + C_t$$

(Eq. 1)

Here, L describes the loop feature (the feedback feature 41) at time t, d denotes a decay factor representing the desorption of previously adsorbed molecules and $C_t$ describes the concentration estimation at time t. In examples, the decay factor may be determined on the basis of an exponential decay. The term "online computation" may optionally refer to a computation, in which a value of the feedback feature is recursively replaced by a value determined on a previous value.

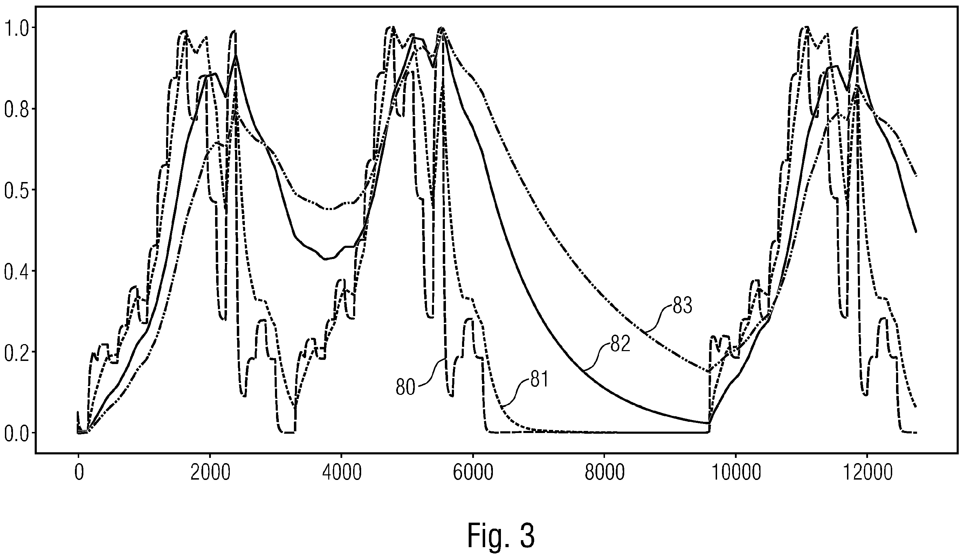

Fig. 3 shows the normalized development of the loop feature 41 over time for different decay factors (curve 81: fast decay, curve 82: medium decay, curve 83: slow decay in comparison with the ground-truth 80 (i.e. the real concentration) of an experimental concentration profile. The decay factor may describe the main parameter in the feature engineering process and is ought to be estimated in a way that it is as much in line with the desorption rate as possible. This could for example be done with specified experiments or with simulations based on a system level model.

By choosing a feasible parameter for the feature calculation in the feedback loop, the approach could enhance the network's concentration prediction accuracy and make it more robust against the exposure to high gas concentrations and hence make it more stable. Additionally, the mechanism described here is rather simple and does not require a lot of additional computational resources.

A determination of the feedback feature 41 according to equation 1 may be particularly beneficial, if the measurements of the concentration are taken at a constant sampling rate, so that a time interval between the measurements is constant. Thus, the time interval between the measurements may be considered in the decay rate d, thus providing for a low implementation effort and a low number of computational operation.

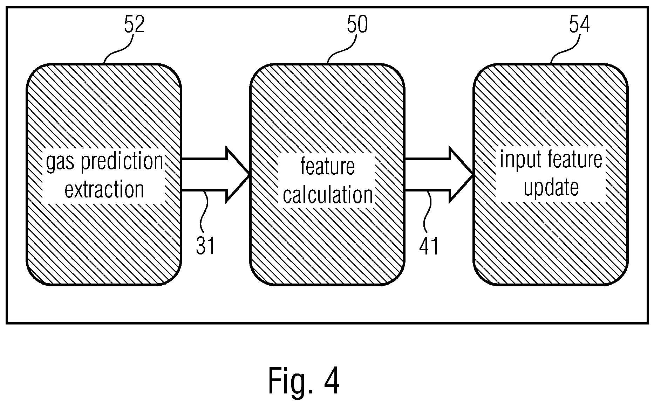

Further examples of implementations of the determination of the feedback feature 41 are described with respect to Fig. 4.

Fig. 4 illustrates a flow chart of a feedback loop procedure according to an example as it may be implemented by the feedback module 40 of Fig. 2. In a step 52, one or more calibrated measurement values to be used in the determination of the feedback feature 41 are extracted or gathered. For example, if the calibrated measurement value 31 is used, the value may be retrieved from the calibration unit 32 in step 52. In examples, in which several previous calibrated measurement values are used, a buffer for storing the previous values may be updated in step 52. The calibrated measurement value 31 may also be referred to as gas prediction or concentration prediction, and consequently, step 52 may be referred to as gas prediction extraction. In a step 50, the feature calculation, the feedback feature 41 is determined on the basis of the calibrated measurement value 31 and optionally further previous calibrated measurement values. In step 54, the feedback feature 41 is provided to the machine learning model 34 as an input feature. In other words, the feedback feature used as input feature by the machine learning model 34 is updated in step 54, e.g., replaced by the feedback feature 41 determined in step 50.

For the implementation of the feedback loop architecture shown in Fig. 4, different design choices may provide for adaptivity and complexity in computation requirements, especially important for low cost and low power systems.

According to one example of step 52 of the gas prediction extraction, the predictions, that is, the calibrated measurement values to an online feature calculation algorithm which updates the previous value or the previous values with respect to the new prediction or the new predictions. This example may be referred to as online feature implementation. An online feature scenario could be achieved by using the following equation for feature calculation:

$$L_t = d \cdot L_{t-1} + \hat{c}_t$$

where the concentration $C_t$ in Eq.1 is replaced by the concentration estimate $\hat{c}_t$. The advantage of the online feature prediction is that it is easier to calculate by simultaneously using less memory.

According to an alternative example of step 52 of the gas prediction extraction, an array of previous predictions over a specified time frame is buffered and the feedback feature 41 is calculated over this time frame, that is, on the basis of the calibrated measurement values of the buffered array. This implementation may be referred to as prediction buffering implementation. The prediction buffering implementation may be beneficial in the case of complex physical properties of the sensing material as it may provide for a more complex feature which better replicates the sensor desorption behavior. For the buffering option, an exponentially-weighted approach would be feasible, as described by the following equation:

$$L_t = \sum_{t \in \{0, t_{i-1}\}} e^{-\lambda(t_{i-1} - t)} \hat{c}_t$$

Here, i describes the feature in the i-th time step, $t_{i-1}$ - $t$ hence describes the time difference of the previous time step to the time iterative t. $\hat{c}_t$ refers to the concentration estimate at time point t. $\lambda$ is a parameter accounting for the exponential decay. Therefore, the impact of concentrations that were measured in the long past is rather low, considering that most of the molecules that adsorbed at that time might already have desorbed, whereas the previous concentrations have a high impact on the feature.

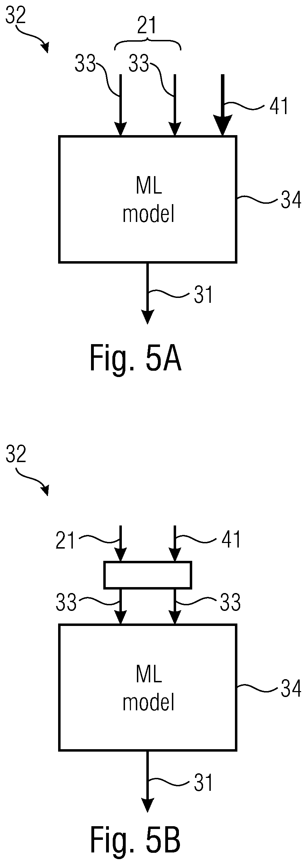

Fig. 5A illustrates an example of the calibration unit 32 as it may optionally be implemented in the sensing device 10 described with respect to Figs. 2 to 4. According to the example of Fig. 5A, the machine learning model 34 receives the measurement information 21 as input features 33. Further, the machine learning model 34 generates at least one output feature of the machine learning model 34 on the basis of the input features. The signal calibration unit 32 obtains the calibrated measurement value 31 as the output feature of the machine learning model 34.

In other words, the machine learning model 34 may generate at least one output feature on the basis of a plurality of input features 33, wherein the input features 33 represent, or are based on, the measurement information 21, and the output feature is the calibrated measurement value 31.

As explained with respect to Fig. 2, the input features 33 may represent different types of measured data measured by the measurement module 20.

According to an example, the machine learning model 34 is configured for using the feedback feature 41 as an input feature for the determination of the subsequent calibrated measurement value 31'.

Using the feedback feature 41 as an input feature for the machine learning model 34 has the advantage that the contribution of the feedback feature 41 for the determination of the calibrated measurement value 31 may be trained together with the model of the machine learning model 34.

Fig. 5B illustrates an example of the calibration unit 32 which is an alternative implementation to the example shown in Fig. 5A. According to the example of Fig. 5B, the calibration unit 32 is configured for adapting the subsequent measurement information 21' in dependence on the feedback feature 41 so as to obtain the input features 33 for the determination of the subsequent calibrated measurement value 31'.

Thus, the example of Fig. 5B may differ from the example of Fig. 5A in that the feedback feature 41, instead of being provided as an input feature to the machine learning model 34, is used for adapting the subsequent measurement information 21'. In other words, the subsequent measurement information 21' may be recalibrated using the feedback feature 41. Compared to the implementation of Fig. 5A, the implementation of Fig. 5B has the advantage, that the machine learning model 34 may be implemented independent of the feedback loop, as the feedback feature is no input feature of the machine learning model. Thus, the example of Fig. 5B may allow for a simple implementation.

Referring to the sensing device 10 described with respect to Figs. 2 to 4, and in particular to the implementation of the calibration unit 32 of Fig. 5A and 5B, the machine learning model 34 may be a neural network. For example, the neural network may be one out of a recurrent neural network (RNN), a feed forward neural network (FFNN), or a convolution neural network (CNN).

For example, the neural network may have multiple layers including an input layer and an output layer, the input layer being configured for receiving the input features 33, and the output layer being configured for providing the at least one output feature 31.

The implementation of the machine learning model 34 as a recurrent neural network may be particularly beneficial in combination with examples of the measurement module 20 which rely on an adsorption based sensing principle, such as the sensing module 22, as the recurrent neural network may consider previous measurement information in the determination of a current calibrated measurement value. Thus, the recurrent neural network may account for the fact that an adsorption based sensing principle current measurement information may depend on previous concentration levels of the target gas. The implementation of the machine learning model 34 as a recurrent neural network is particularly beneficial in combination with the implementation of the feedback feature 41, as the contribution of recurrent features of the recurrent neural network in the determination of the calibrated measurement value 31', and the contribution of the feedback feature 41 in the determination of the calibrated measurement value 31' may follow different time constants. For example, the recurrent characteristics of the recurrent neural network may have a rather short time constant, so that the recurrent features of the recurrent neural networks may provide for a contribution of previous measurement information from a short time interval before the measurement of the current measurement information 21', wherein the feedback feature 41 may provide a contribution of measurement information from a longer time interval before the measurement of the current measurement information 21'.

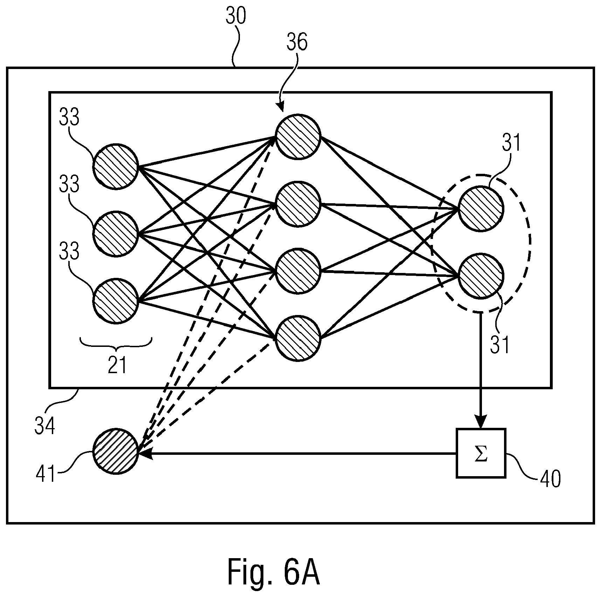

Fig. 6A illustrates an example of the signal calibration module 30, according to which the machine learning model 34 is implemented as a neural network. According to the example of Fig. 6A, the neural network 34 receives the measurement information 21 as input features 33 and further receives the feedback feature 41 as an additional input feature. Thus, the implementation of Fig. 6A may be in accordance with the calibration unit 32 of Fig. 5A. According to Fig. 6A, the neural network 34 may determine a set of intermediate features 36 on the basis of the input features 33 and the feedback feature 41. Further, the neural network may determine the output feature, i.e. the one or more calibrated measurement values 31 on the basis of the intermediate features 36. It is noted, that although Fig. 6A illustrates only one intermediate step between the input and the output, the neural network may have an arbitrary number of hidden layers. The feedback module 40 determines, on the basis of the at least one calibrated measurement values 31 the feedback feature 41, as described with respect to Fig. 2 to 4, for example, on the basis of a weighted sum, e.g., as described with respect to the feature calculation step 50 of Fig. 4.

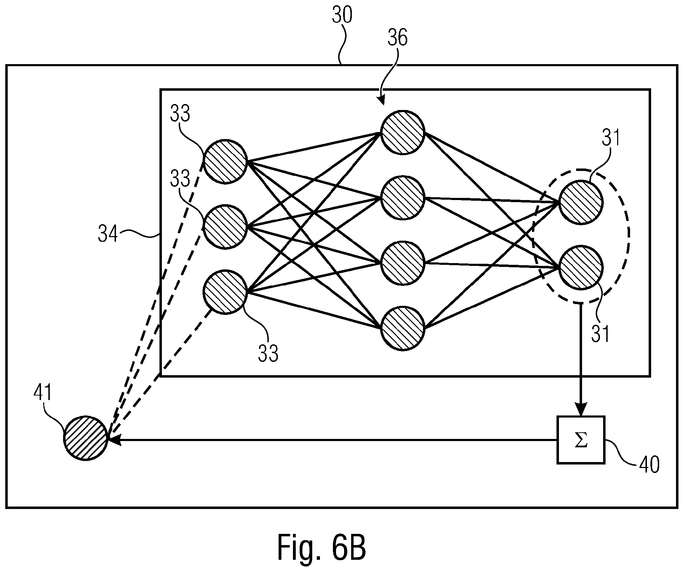

Fig. 6B illustrates another example of the signal calibration module 30 which is an alternative implementation compared to the implementation shown in Fig. 6A. The implementation of Fig. 6B may be in accordance with the calibration unit 32 of Fig. 5B. According to the example of Fig. 6B, the machine learning module 34 is implemented as a neural network, as described with respect to Fig. 6A, however, according to the example of Fig. 6B, the feedback feature 41 may be used for recalibration or adaption of the input features 33 instead of providing the feedback feature 41 as an input feature to the neural network 34. The adaption or recalibration of the input features 33 may be performed prior to providing the input features 33 to the neural network 34, or may be implemented as an additional layer of the neural network 34.

In other words, an alternative to the approach described with respect to Fig. 6A is to use the feedback feature 41 for input vector standardization in the input layer. The input vector may refer to the entity of input features 33. Standardization of the input vector may be achieved by introducing a normalization layer using the feedback feature 41 as input, the normalization layer being configured for renormalizing the input features 33 to compensate directly for drift related base line shifts. In other words, according to the implementation of Fig. 6B, a recalibration step may be performed before the input layer of the neural network model 34. As described with respect to Fig. 6A, the feedback module may be implemented as described with respect to Fig. 2 to 4, for example, on the basis of a weighted sum, e.g., as described with respect to the feature calculation step 50 of Fig. 4.

According to examples, the measurement model 20 of the sensing device 10 as described with respect to Figs. 2 to 6, comprises a plurality of chemoresistive sensing units 24, for example, as described with respect to Fig. 1. Each of the chemoresistive units 24 is configured for providing a respective measurement signal. According to these examples, the measurement information 21, 21' is based on the measurement signals provided by the sensing units 24.

Having a plurality of sensing units 24, e.g. being sensitive to different gasses, may make the determination of the calibrated measurement value 31 particularly robust in the present of gas mixtures and/or humidity.

As described with respect to Fig. 1, the plurality of chemoresistive sensing units 24 may be sensitive to a plurality of target gasses. Thus, in examples, in which the measurement module 20 provides measurement information 21 being based on a plurality of measurement signals of respective sensing units 24, the machine learning model 34 may determine, on the basis of the measurement information 21 respective calibrated measurement values 31 for concentrations of the target gasses. In other words, the machine learning model 34 may determine, for each of the target gasses of the measurement module 20, a calibrated measurement value 31 for the concentration of the target gas. Thus, according to these examples, the measurement information 21 of a measurement may comprise information about the measurement signals at the time of the measurement of the measurement information 21. Consequently, the input features 33 as described with respect to Fig. 5 and 6 may include input features which represent the plurality of measurement signals of the sensing unit 24.

According to examples, in which the sensing device is sensitive to a plurality of target gasses, and the machine learning model 34 determines respective calibrated measurement values 31 for the target gasses, the feedback module 40 may determine, for each of the target gasses, a feedback feature 41 on the basis of the calibrated measurement value 31 determined for the target gas. Accordingly, the calibration unit 32 may determine the subsequent calibrated measurement values 31' for the target gasses using the feedback features 41.

In other words, the feedback module 40 may determine one feedback feature 41 for each target gas. Thus, referring to Figs. 2, 4, 5A and 6A, the machine learning model 34 may, for each measurement, e.g. the measurement of the subsequent measurement information 21', determine a plurality of calibrated measurement values 31', on the basis of the corresponding measurement information, e.g. the subsequent measurement information 21', and on the basis of a plurality of feedback features 41. Thus, the machine learning model 24 may receive a plurality of feedback features 41 as input features. Accordingly, the machine learning module 34 may determine the respective calibrated measurement values 31' for the plurality of target gasses on the basis of the input features 33 of the measurement information 21' and the feedback features 41.

The determination of individual feedback features 41 for the target gasses allows to adapt the determination of the respective feedback features 41 to different dynamics in the sensing of the respective target gas, for example, to different desorption rates of different target gasses. In other words, determining one feedback feature 41 for every target gas measured by the sensor provides for a high flexibility in accounting for different desorption rates.

According to alternative examples for the case of a plurality of target gasses, the feedback module 40 determines the feedback feature 41 on the basis of the calibrated measurement values 31 determined for the plurality of target gasses.

That is, for example, the feedback module 40 may determine one feedback feature 41 on the basis of the calibrated measurement values 31 determined for the plurality of target gasses. For example, the feedback module 40 may use a weighted sum for combining the calibrated measurement values, and optionally a previous feedback feature. Alternatively, the feedback module 40 may use a further machine learning model or neural network for determining the feedback feature 41. Determining a common feedback feature 41 for the plurality of calibrated measurement values for the plurality of target gasses has the advantage of a simple implementation and smaller calculation and buffering effort. For example, in examples, in which the feedback feature 41 represents a drift related side occupancy of the sensing units 24 using one feedback feature combining contributions of the several predictions, i.e., several calibrated measurement values 31, may be a reasonable trait of between implementation effort and benefit. In particular, in this case, using a neural network for performing determination of the feedback feature may be beneficial.

It is noted, that also combinations of the two above described examples are possible. That is, the feedback module 40 may determine a number of feedback features, which number does not necessarily correspond to the number of target gases or calibrated measurement values. For example, the calibrated measurement values for target gases having similar dynamics may be combined. Thus, a beneficial trade-off between low memory requirements and a high adaptivity to the dynamics of the target gases may be achieved.

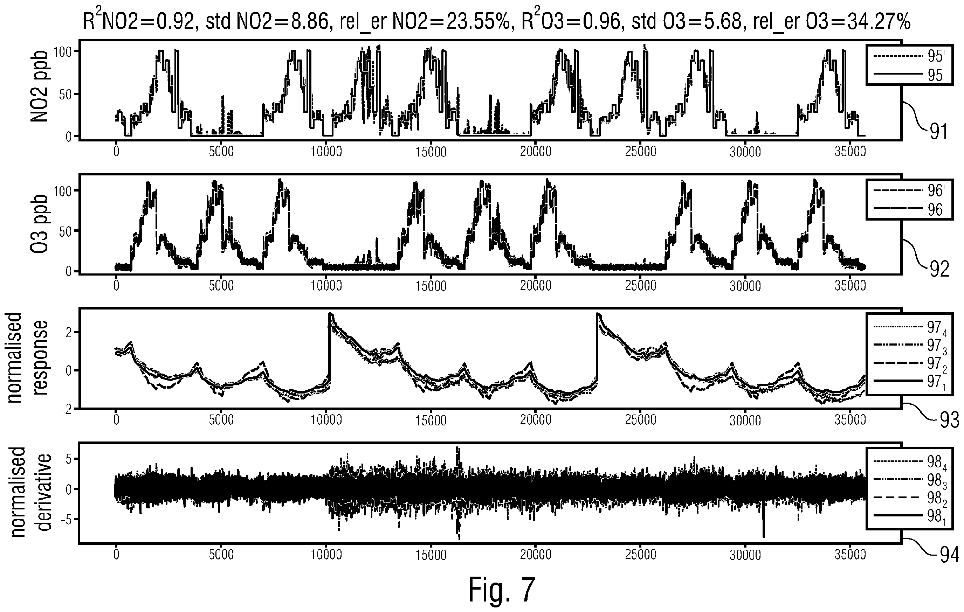

Fig. 7 illustrates the accuracy of an example of the determination of the calibrated measurement values 31 according to an example of the disclosure. Diagram 91 of Fig. 7 shows a comparison between a true concentration profile 95 of $NO_2$ and the prediction 95' (i.e. a sequence of calibrated measurement values 95') determined by an example of the sensing device 10. Diagram 92 shows a comparison between a true concentration profile 96 of $O_3$ and a prediction 96' of $O_3$ concentrations. Diagram 93 shows normalized responses $97_1$, $97_2$, $97_3$, $97_4$ of sensing units 24, which may be examples of the measurement signals based on which the measurement information 21, 21' may be obtained. Diagram 94 shows normalized derivatives $98_1$, $98_2$, $98_3$, $98_4$ of the normalized responses of diagram 93. Normalized derivatives may also be provided as part of the measurement information 21, 21'.

In the example of Fig. 7, in order to demonstrate the effectiveness of the back feature, the ground truth signal has been taken for determining the feedback feature 41. Therefore, the input concentration values have been used for feedback loop feature calculation using the exponentially-weighted buffering method as described in the following equation:

$$L_i = \sum_{t \in \{0, t_{i-1}\}} e^{-\lambda(t_{i-1} - t)} \hat{c}_t$$

There was one loop feature for each of the two measuring gases to account differently for the type of gas. The resulting network was trained and tested on a test profile of concentrations containing both gas types and varying in the range of concentrations. The results showing the concrete gas predictions of the test data set is shown in Fig. 7. It can be seen that the algorithm predictions are well in-line with the ground-truth of the concentration values except for some small overestimation of the $NO_2$ predictions when only $O_3$ is present. For the Ozone predictions, this effect can only slightly be derived in one part of the test profile. The R2 scores show a high correlation between the predicted and the estimated concentration values.

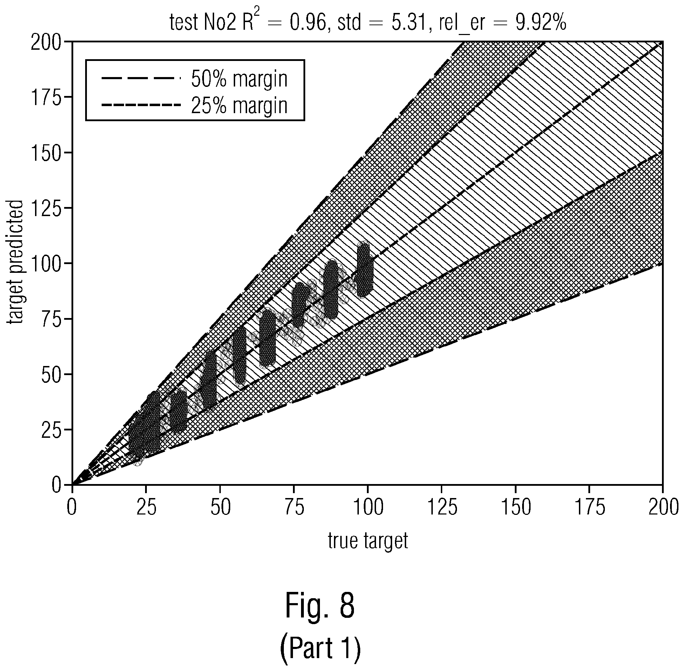

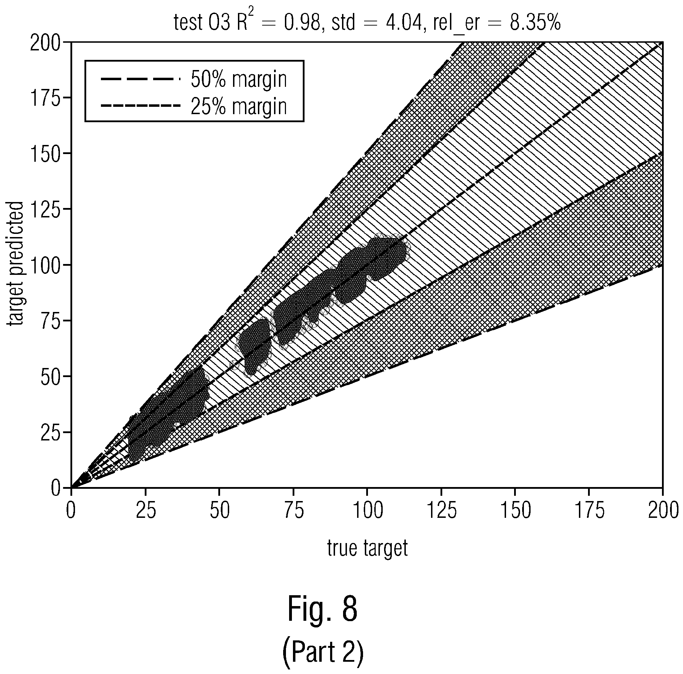

Fig. 8 illustrates scores for the best 90% of the predictions for the two gas types of Fig. 7. The relative error is comparably low and the majority of data points are within the 25% relative error or margin.

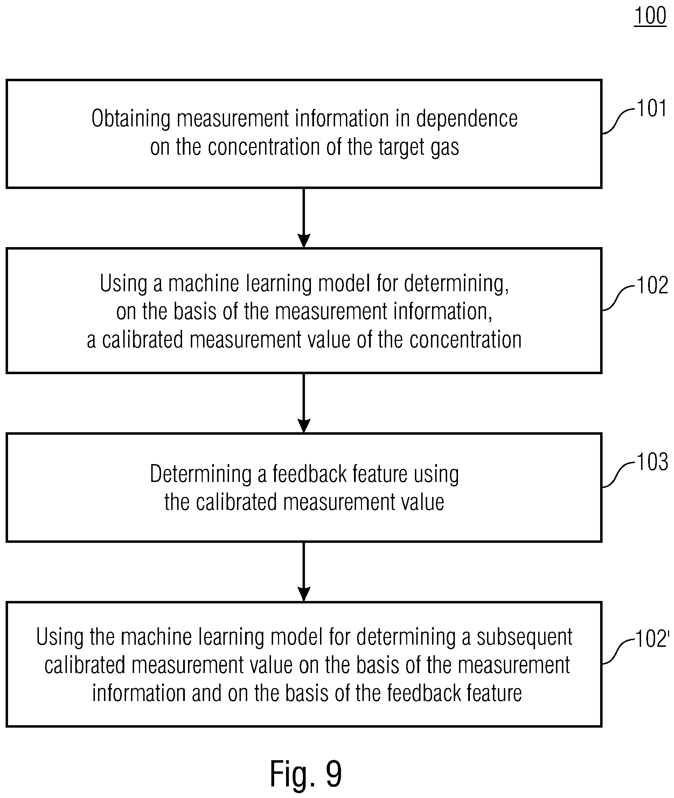

Fig. 9 shows a flow chart of a method 100 for determining a calibrated measurement value 31 of a concentration of target gas. The method 100 comprises a step 101 of obtaining measurement information in dependence on the concentration of the target gas. For example, step 101 may be performed by the measurement model 20. The method 100 further comprises a step 102 of using a machine learning model 34 for determining, on the basis of the measurement information, the calibrated measurement value 31 of the concentration. The method 100 further comprises a step 103 of determining a feedback feature 41 using the calibrated measurement value 31. For example, step 103 may be performed by the feedback module 40. For example, the step 103 may correspond to step 50 of Fig. 4. The method 100 comprises a step 102' of using 102' the machine learning model 34 for determining a subsequent calibrated measurement value 31' on the basis of the measurement information and on the basis of the feedback feature 41.

For example, step 102' may correspond to step 102, steps 102 and 102' representing the determination of respective calibrated measurement values 31 and 31' for subsequent measurements.

For example, step 101 comprises obtaining the measurement information 21 about a measurement of the concentration of the target gas and step 102 comprises using the machine learning model 34 for determining, on the basis of the measurement information 21, the calibrated measurement value 31. According to this example, step 102' comprises using the machine learning model 34 for determining the subsequent calibrated measurement value 31' on the basis of measurement information 21' about a subsequent measurement of the concentration of the target gas and on the basis of the feedback feature 41.

According to examples, step 102 comprises using the machine learning model 34 for generating at least one output feature of the machine learning model 34 on the basis of a plurality of input features of the machine learning model 34. According to these examples, the method comprises providing the measurement information 21 as input features 33 to the machine learning model 34, and obtaining the calibrated measurement value 31 as an output feature of the machine learning model 34.

According to examples, step 102 comprises using the feedback feature 41 as an input feature of the machine learning model 34 for the determination of the subsequent calibrated measurement value 31'.

According to examples, the method comprises adapting this measurement information 21' about the subsequent measurement independence on the feedback feature 41 so as to obtain the input features 33 for the determination of a subsequent calibrated measurement value 31'.

According to examples, step 103 comprises determining the feedback feature 41 for the determination of the subsequent calibrated measurement value 31' on the basis of the calibrated measurement value 31 and on the basis of one or more previously determined calibrated measurement values.

According to examples, step 103 comprises determined the feedback feature 41 for the determination of the subsequent calibrated measurement value 31 by updating a previously determined feedback feature using the calibrated measurement value 31, wherein the previously determined feedback feature is determined on the basis of previous calibrated measurement values.

According to examples, the step 103 comprises determining the feedback feature 41 on the basis of a weighted sum of the calibrated measurement value and one or more previously determined calibrated measurement values.

According to examples, step 101 comprises determining the measurement information using at least one chemoresistive sensing unit which is sensitive to the target gas. According to these examples, the weights of the weighted sum are adapted to a desorption rate of molecules of the target gas adsorbed at the surface region of the sensing unit.

According to examples, the machine learning model 34 is a recurrent neural network, or a feed forward neural network, or a convolutional neural network.

According to examples, step 101 comprises obtaining the measurement information and subsequent measurement information from a sequence of measurements of the concentration. According to these examples, the calibrated measurement value 31 and the subsequent measurement value 31' are part of the sequence of calibrated measurement values, and step 102 comprises determining the calibrated measurement values on the basis of measurement information resulting from respective measurements of the sequence of measurements. According to these examples, the step 103 comprises determining the feedback feature 41 for the determination of one of the calibrated measurement values by recursively updating the feedback feature 41 using the previous one of the calibrated measurement values 31.

According to examples, step 101 comprises obtaining the measurement information 21, 21' on the basis of respective measurement signals provided by a plurality of chemoresistive sensing units 24.

According to examples, the target gas is part of a plurality of target gasses of the sensing device and step 102 comprises using the machine learning model 34 for determining, on the basis of the measurement information 21, respective calibrated measurement values 31 for concentrations of the target gasses. According to these examples, step 103 comprises determining, for each of the target gasses, a feedback feature 41 on the basis of the calibrated measurement value 31 determined for the target gas. Further, step 102 comprises determining the subsequent calibrated measurement values 31' for th target gasses using the feedback features 41.

According to examples, the target gas is part of a plurality of target gasses of the sensing device. According to these examples, the step 102 comprises using the machine learning model 34 for determining, on the basis of the measurement information 21, respective calibrated measurement values 31 of concentrations of the target gasses. According to these examples, step 103 comprises determining the feedback feature 41 on the basis of the calibrated measurement values, and determining subsequent calibrated measurement values. Further, according to these examples, step 102 comprises, in the determination of the subsequent calibrated measurement values 31', using the feedback feature 41.

According to examples, a computer program is for implementing the method 100 when being executed on a computer or signal processor.

In the following, further examples of the present disclosure are described.

According to examples, a sensing device 10 for sensing a concentration of a target gas, comprises: a measurement module 20 configured for providing measurement information 21 about a measurement of the concentration; and a signal calibration module 30 configured for using a machine learning model 34 for determining, on the basis of the measurement information 21, a calibrated measurement value 31 of the concentration, further configured for determining a feedback feature 41 using the calibrated measurement value 31, and further configured for using the machine learning model 34 for determining a subsequent calibrated measurement value 31' on the basis of subsequent measurement information 21' about a subsequent measurement of the concentration and on the basis of the feedback feature 41.

According to examples, the machine learning model 34 is configured for generating at least one output feature of the machine learning model 34 on the basis of a plurality of input features of the machine learning model 34; and the signal calibration module 30 is configured for providing the measurement information 21 as input features 33 to the machine learning model 34, and for obtaining the calibrated measurement value 31 as an output feature of the machine learning model 34.

According to examples, the machine learning model 34 is configured for using the feedback feature 41 as an input feature for the determination of the subsequent calibrated measurement value 31'.

According to examples, the signal calibration module 30 is configured for adapting the subsequent measurement information 21' in dependence on the feedback feature 41 so as to obtain the input features 33 for the determination of the subsequent calibrated measurement value 31'.

According to examples, the signal calibration module 30 is configured for determining the feedback feature 41 for the determination of the subsequent calibrated measurement value 31' on the basis of the calibrated measurement value 31 and on the basis of one or more previously determined calibrated measurement values.

According to examples, the signal calibration module 30 is configured for determining the feedback feature 41 for the determination of the subsequent calibrated measurement value 31' by updating a previously determined feedback feature using the calibrated measurement value 31, wherein the previously determined feedback feature is determined on the basis of previous calibrated measurement values.

According to examples, the signal calibration module 30 is configured for determining the feedback feature 41 on the basis of a weighted sum of the calibrated measurement value 31 and one or more previously determined calibrated measurement values.

According to examples, wherein the measurement module 20 comprises at least one chemoresistive sensing unit 24 which is sensitive to the target gas, and wherein weights of the weighted sum are adapted to a desorption rate of molecules of the target gas adsorbed at a surface region 25 of the sensing unit 24.

According to examples, the machine learning model 34 is a recurrent neural network, a feed-forward neural network, or a convolutional neural network.

According to examples, the measurement information 21 and the subsequent measurement information 21' result from a sequence of measurements of the concentration. Further, the calibrated measurement value 31 and the subsequent measurement value 31' are part of a sequence of calibrated measurement values, and the signal calibration module 30 is configured for determining the calibrated measurement values on the basis of measurement information resulting from respective measurements of the sequence of measurements. Further, the signal calibration module 30 is configured for determining the feedback feature 41 for the determination of one of the calibrated measurement values by recursively updating the feedback feature 41 using the previous one of the calibrated measurement values.

According to examples, the measurement module 20 comprises a plurality of chemoresistive sensing units 24, each of which is configured for providing a respective measurement signal, and wherein the measurement information 21 is based on the measurement signals provided by the sensing units.

According to examples, the target gas is part of a plurality of target gases of the sensing device, and the signal calibration module 30 is configured for
using the machine learning model 34 for determining, on the basis of the measurement information 21, respective calibrated measurement values for concentrations of the target gases,
determining, for each of the target gases, a feedback feature 41 on the basis of the calibrated measurement value 31 determined for the target gas, and
determining subsequent calibrated measurement values 31' for the target gases using the feedback features 41.

According to examples, the target gas is part of a plurality of target gases of the sensing device, and the signal calibration module 30 is configured for
using the machine learning model 34 for determining, on the basis of the measurement information 21, respective calibrated measurement values 31 of concentrations of the target gases,
determining the feedback feature 41 on the basis of the calibrated measurement values 31, and
determining subsequent calibrated measurement values 31' for the target gases using the feedback feature 41.

Although some aspects have been described as features in the context of an apparatus it is clear that such a description may also be regarded as a description of corresponding features of a method. Although some aspects have been described as features in the context of a method, it is clear that such a description may also be regarded as a description of corresponding features concerning the functionality of an apparatus.

Some or all of the method steps may be executed by (or using) a hardware apparatus, like for example, a microprocessor, a programmable computer or an electronic circuit. In some examples, one or more of the most important method steps may be executed by such an apparatus.

Depending on certain implementation requirements, examples of the invention can be implemented in hardware or in software or at least partially in hardware or at least partially in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some examples according to the invention comprise a data carrier having electronically readable control signals, which are capable of cooperating with a programmable computer system, such that one of the methods described herein is performed.

Generally, examples of the present invention can be implemented as a computer program product with a program code, the program code being operative for performing one of the methods when the computer program product runs on a computer. The program code may for example be stored on a machine readable carrier.

Other examples comprise the computer program for performing one of the methods described herein, stored on a machine readable carrier.

In other words, an example of the disclosed method is, therefore, a computer program having a program code for performing one of the methods described herein, when the computer program runs on a computer.

A further example of the disclosed methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitory.

A further example of the disclosed method is, therefore, a data stream or a sequence of signals representing the computer program for performing one of the methods described herein. The data stream or the sequence of signals may for example be configured to be transferred via a data communication connection, for example via the Internet.

A further example comprises a processing means, for example a computer, or a programmable logic device, configured to or adapted to perform one of the methods described herein.

A further example comprises a computer having installed thereon the computer program for performing one of the methods described herein.

A further example according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some examples, a programmable logic device (for example a field programmable gate array) may be used to perform some or all of the functionalities of the methods described herein. In some examples, a field programmable gate array may cooperate with a microprocessor in order to perform one of the methods described herein. Generally, the methods are preferably performed by any hardware apparatus.

The apparatus described herein may be implemented using a hardware apparatus, or using a computer, or using a combination of a hardware apparatus and a computer.

The methods described herein may be performed using a hardware apparatus, or using a computer, or using a combination of a hardware apparatus and a computer.

In the foregoing Detailed Description, it can be seen that various features are grouped together in examples for the purpose of streamlining the disclosure. This method of disclosure is not to be interpreted as reflecting an intention that the claimed examples require more features than are expressly recited in each claim. Rather, as the following claims reflect, subject matter may lie in less than all features of a single disclosed example. Thus the following claims are hereby incorporated into the Detailed Description, where each claim may stand on its own as a separate example. While each claim may stand on its own as a separate example, it is to be noted that, although a dependent claim may refer in the claims to a specific combination with one or more other claims, other examples may also include a combination of the dependent claim with the subject matter of each other dependent claim or a combination of each feature with other dependent or independent claims. Such combinations are proposed herein unless it is stated that a specific combination is not intended. Furthermore, it is intended to include also features of a claim to any other independent claim even if this claim is not directly made dependent to the independent claim.

The above described examples are merely illustrative for the principles of the present disclosure. It is understood that modifications and variations of the arrangements and the details described herein will be apparent to others skilled in the art. It is the intent, therefore, to be limited only by the scope of the pending patent claims and not by the specific details presented by way of description and explanation of the examples herein.

## Claims

1. Sensing device (10) for sensing a concentration of a target gas, comprising:

   a measurement module (20) configured for providing measurement information (21) about a measurement of the concentration,
   a signal calibration module (30) configured for

   using a machine learning model (34) for determining, on the basis of the measurement information (21), a calibrated measurement value (31) of the concentration,
   determining a feedback feature (41) using the calibrated measurement value (31), and using the machine learning model (34) for determining a subsequent calibrated measurement value (31') on the basis of subsequent measurement information (21') about a subsequent measurement of the concentration and on the basis of the feedback feature (41).

2. Sensing device (10) according to claim 1, wherein the machine learning model (34) is configured for generating at least one output feature of the machine learning model (34) on the basis of a plurality of input features of the machine learning model (34), and wherein the signal calibration module (30) is configured for providing the measurement information (21) as input features (33) to the machine learning model (34), and for obtaining the calibrated measurement value (31) as an output feature of the machine learning model (34).

3. Sensing device (10) according to claim 2, wherein the machine learning model (34) is configured for using the feedback feature (41) as an input feature for the determination of the subsequent calibrated measurement value (31').

4. Sensing device (10) according to claim 2, wherein the signal calibration module (30) is configured for adapting the subsequent measurement information (21') in dependence on the feedback feature (41) so as to obtain the input features (33) for the determination of the subsequent calibrated measurement value (31').

5. Sensing device (10) according to any of the preceding claims, wherein the signal calibration module (30) is configured for determining the feedback feature (41) for the determination of the subsequent calibrated measurement value (31') on the basis of the calibrated measurement value (31) and on the basis of one or more previously determined calibrated measurement values.

6. Sensing device (10) according to claim 5, wherein the signal calibration module (30) is configured for determining the feedback feature (41) for the determination of the subsequent calibrated measurement value (31') by updating a previously determined feedback feature using the calibrated measurement value (31), wherein the previously determined feedback feature is determined on the basis of previous calibrated measurement values.

7. Sensing device (10) according to any of the preceding claims, wherein the signal calibration module (30) is configured for determining the feedback feature (41) on the basis of a weighted sum of the calibrated measurement value (31) and one or more previously determined calibrated measurement values.

8. Sensing device (10) according to claim 7, wherein the measurement module (20) comprises at least one chemoresistive sensing unit (24) which is sensitive to the target gas, and wherein weights of the weighted sum are adapted to a desorption rate of molecules of the target gas adsorbed at a surface region (25) of the sensing unit (24).

9. Sensing device (10) according to any of the preceding claims, wherein the machine learning model (34) is a recurrent neural network, a feed-forward neural network, or a convolutional neural network.

10. Sensing device (10) according to any of the preceding claims, wherein the measurement information (21) and the subsequent measurement information (21') result from a sequence of measurements of the concentration,
    wherein the calibrated measurement value (31) and the subsequent measurement value (31') are part of a sequence of calibrated measurement values, wherein the signal calibration module (30) is configured for determining the calibrated measurement values on the basis of measurement information resulting from respective measurements of the sequence of measurements, and
    wherein the signal calibration module (30) is configured for determining the feedback feature (41) for the determination of one of the calibrated measurement values by recursively updating the feedback feature (41) using the previous one of the calibrated measurement values.

11. Sensing device (10) according to any of the preceding claims, wherein the measurement module (20) comprises a plurality of chemoresistive sensing units

(24), each of which is configured for providing a respective measurement signal, and wherein the measurement information (21) is based on the measurement signals provided by the sensing units.

12. Sensing device (10) according to claim 11, wherein the target gas is part of a plurality of target gases of the sensing device, and wherein the signal calibration module (30) is configured for

using the machine learning model (34) for determining, on the basis of the measurement information (21), respective calibrated measurement values for concentrations of the target gases,
determining, for each of the target gases, a feedback feature (41) on the basis of the calibrated measurement value (31) determined for the target gas, and
determining subsequent calibrated measurement values (31') for the target gases using the feedback features (41).

13. Sensing device (10) according to claim 11, wherein the target gas is part of a plurality of target gases of the sensing device, and wherein the signal calibration module (30) is configured for

using the machine learning model (34) for determining, on the basis of the measurement information (21), respective calibrated measurement values (31) of concentrations of the target gases,
determining the feedback feature (41) on the basis of the calibrated measurement values (31), and
determining subsequent calibrated measurement values (31') for the target gases using the feedback feature (41).

14. Method (100) for determining a calibrated measurement value (31) of a concentration of a target gas, comprising:

obtaining (101) measurement information in dependence on the concentration of the target gas,
using (102) a machine learning model (34) for determining, on the basis of the measurement information, the calibrated measurement value (31) of the concentration,
determining (103) a feedback feature (41) using the calibrated measurement value (31), and
using (102') the machine learning model (34) for determining a subsequent calibrated measurement value (31') on the basis of the measurement information and on the basis of the feedback feature (41).

15. Computer program for implementing the method of claim 14 when being executed on a computer or signal processor.

Fig. 1

Fig. 2

Fig. 3

52

50

54

gas prediction
extraction

feature
calculation

input feature
update

31

41

Fig. 4

32

21

33   33                    41

ML
model                        34

31

Fig. 5A

32

21              41

33              33

ML
model              34

31

Fig. 5B

Fig. 6A

Fig. 6B

Fig. 7

Fig. 8
(Part 1)

test O3 $R^2$ = 0.98, std = 4.04, rel_er = 8.35%

Fig. 8
(Part 2)

100

Obtaining measurement information in dependence
on the concentration of the target gas ⟋─101

Using a machine learning model for determining,
on the basis of the measurement information,
a calibrated measurement value of the concentration ⟋─102

Determining a feedback feature using
the calibrated measurement value ⟋─103

Using the machine learning model for determining a subsequent
calibrated measurement value on the basis of the measurement
information and on the basis of the feedback feature ⟋─102'

Fig. 9

**EP 4 080 202 A1**

## EUROPEAN SEARCH REPORT

Application Number

EP 21 16 9651

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG QINGFENG ET AL: "Time Series Prediction of E-nose Sensor Drift Based on Deep Recurrent Neural Network", 2019 CHINESE CONTROL CONFERENCE (CCC), TECHNICAL COMMITTEE ON CONTROL THEORY, CHINESE ASSOCIATION OF AUTOMATION, 27 July 2019 (2019-07-27), pages 3479-3484, XP033632413, DOI: 10.23919/CHICC.2019.8866168 [retrieved on 2019-10-10] * figure 5 * * sections 3.2, 5 * * page 3479, left-hand column, lines 3-12 * * abstract * * section 1, first paragraph * | 1-15 | INV. G01N27/12 G01N33/00 |
| X | HOLMBERG M ET AL: "Drift counteraction in odour recognition applications: Lifelong calibration method", SENSORS AND ACTUATORS B: CHEMICAL, ELSEVIER BV, NL, vol. 42, no. 3, 1 August 1997 (1997-08-01), pages 185-194, XP004100264, ISSN: 0925-4005, DOI: 10.1016/S0925-4005(97)80335-8 * figure 1 * * page 187, left-hand column, lines 1-3, 11-13 * * page 187, right-hand column, lines 14-15 * | 1-6,10, 14,15 | TECHNICAL FIELDS SEARCHED (IPC) G01N |
| A | US 2020/371076 A1 (KOENIG MATTHIAS [DE]) 26 November 2020 (2020-11-26) * figures 1-3 * * paragraphs [0027], [0048] - [0058] * | 1-15 | |

-/--

The present search report has been drawn up for all claims

| Place of search | Date of completion of the search | Examiner |
|---|---|---|
| Munich | 28 September 2021 | Kratz, Dorothee |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 21 16 9651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2019/128203 A1 (HEBEI SAILHERO ENV PROT HIGH TECH CO LTD [CN]) 4 July 2019 (2019-07-04) * paragraphs [0038] - [0044] * ----- | 1-15 | |
| A | CN 106 405 007 A (HEBEI SAILHERO ENV PROT HIGH-TECH CO LTD) 15 February 2017 (2017-02-15) * figure 1 * * paragraphs [0033] - [0035] * ----- | 1-15 | |

TECHNICAL FIELDS
SEARCHED       (IPC)

The present search report has been drawn up for all claims

| Place of search | Date of completion of the search | Examiner |
|---|---|---|
| Munich | 28 September 2021 | Kratz, Dorothee |

## ANNEX TO THE EUROPEAN SEARCH REPORT
## ON EUROPEAN PATENT APPLICATION NO.

EP 21 16 9651

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2021

| Patent document cited in search report | | Publication date | Patent family member(s) | | Publication date |
|---|---|---|---|---|---|
| US 2020371076 | A1 | 26-11-2020 | DE 102019113539 A1<br>US    2020371076 A1 | | 26-11-2020<br>26-11-2020 |
| WO 2019128203 | A1 | 04-07-2019 | CN     107966534 A<br>WO    2019128203 A1 | | 27-04-2018<br>04-07-2019 |
| CN 106405007 | A | 15-02-2017 | NONE | | |

EPO FORM P0459

For more details about this annex : see Official Journal of the European Patent Office, No. 12/82